# EUROPEAN PATENT APPLICATION

(11) **EP 0 676 238 A2**
(43) Date of publication of application: **11.10.1995**
(21) Application number: 95302244.9
(22) Date of filing: 04.04.1995
(51) Int. Cl.: B01J 31/18, C07C 29/132, C09B 47/04

(54) **Metal-ligand catalysts for oxidation of alkanes and decomposition of organic hydroperoxides**

(30) Priority: 04.04.1994 US 222747
(71) Applicant: SUN COMPANY, INC. (R&M), Philadelphia, PA 19103-1699 (US)
(72) Inventor: Bhinde, Manoj V., Boothwyn, PA 19061 (US); Lyons, James E., Wallingford, PA 19086 (US); Ellis, Paul E., Jr., Downingtown, PA 19335 (US)
(74) Representative: Lewin, John Harvey

(57) **Abstract**

Metallophthalocyanines having hydrocarbyl or other substituents on the benzene rings of the phthalocyanines, and µ-oxo dimers of metallophthalocyanines are highly active catalysts for the decomposition of hydroperoxides and for the partial oxidation of hydrocarbons.

## Description

The Government of the United States of America has rights in this invention pursuant to Cooperative Agreement No. DE-FC21-90MC26029 awarded by the U.S. Department of Energy.

### BACKGROUND

Metallophthalocyanine complexes are known catalysts for the decomposition of hydroperoxides. Sanderson et al U.S. Patents 4,912,366 and 4,912,267 issued March 27, 1990; and 4,922,035 and 4,922,036 issued May 1, 1990 disclose decomposition of t-butyl hydroperoxide (TBHP) dissolved in t-butanol (TBA) over a metallophthalocyanine catalyst modified by imidazole and other modifiers. Sanderson et al U.S. Patent 4,992,602 issued February 12, 1991 discloses partial oxidation of isobutane and distillation of the reaction product to obtain a fraction containing 80 to 90% of t-butylhydroperoxide and 20 to 10% of t-butanol, dissolving that fraction in 3 to 10 parts by weight, based on the weight of the fraction, of benzene, and decomposing the hydroperoxide in the resulting solution with a phthalocyanine decomposition catalyst. Lyons and Ellis U.S. Patent 5,120,886 issued June 9, 1992 discloses and claims decomposition of hydroperoxides by contact with metal ligand catalysts of coordination complexes, including phthalocyanine ligands, in which hydrogen in the phthalocyanine molecule has been substituted with electron-withdrawing elements or groups, for example halogen or nitro or cyano groups.

### DESCRIPTION OF THE INVENTION

The compositions of the invention are phthalocyanines having particularly high activity for the decomposition of hydroperoxides; these phthalocyanines have the following formula: 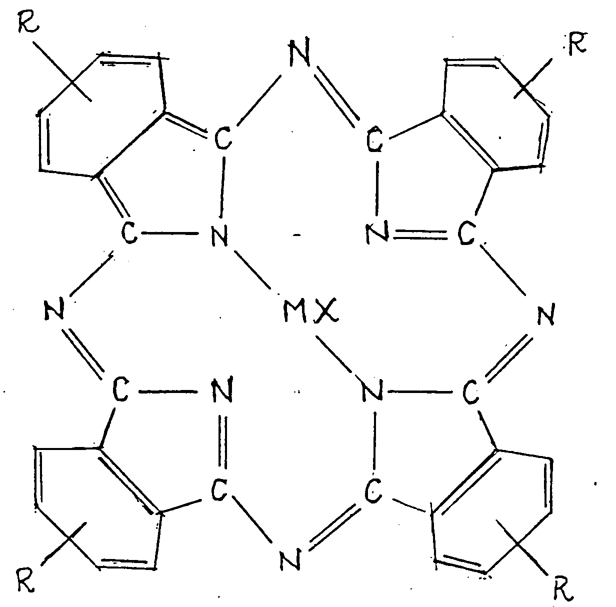
where (1) each R comprises, independently, one to four hydrocarbyl, halocarbyl or halohydrocarbyl groups, or hydrogen, at least one of said R's not being hydrogen, M comprises iron, cobalt, manganese, copper, ruthenium, or chromium, and X comprises halogen, hydroxyl or azide, or (2) said composition comprises a µ-oxo dimer of metallophthalocyanines having said formula, or halometallophthalocyanines.

Compositions as disclosed above wherein each R comprises hydrocarbyl or halocarbyl or halohydrocarbyl are preferred according to the invention. Compositions as disclosed above wherein M comprises iron are also preferred according to the invention.

Preferred µ-oxo dimers according to the invention have the formula: (XPc)M-O-M(XPc), where M comprises iron, cobalt, manganese, ruthenium, copper or chromium, and X comprises perhalo, for example perfluoro, perbromo or perchloro. "Perhalo" indicates complete substitution of halogen for hydrogen, or as near complete such substitution as reasonably obtainable.

The hydrocarbyl, halocarbyl or halohydrocarbyl group represented by R preferably has 1 to 8 carbon atoms.

Examples of R groups in the above compositions are alkyl groups such as methyl, t-butyl and the like, haloalkyl groups such as trifluoromethyl, trichloromethyl and the like, and halohydroalkyl groups such as monofluoromethyl, dichloroethyl groups, and the like.

### HYDROPEROXIDES

Hydroperoxides whose decomposition may be catalyzed by the compositions of the invention include compounds having the formula ROOH where R is an organic radical, typically a straight or branched chain alkyl group or cycloalkyl group containing 2 to 15 carbon atoms, an aryl group such as a monocyclic or polycyclic group in which the cyclic groups may optionally be substituted with one or more substituents inert to the decomposition reaction, such as alkyl or alkoxy, containing 1 to 7 carbon atoms, nitro, carboxyl or carboxyl ester containing up to 15 carbon atoms and a halogen atom such as chloride, bromide or an alkaryl group in which the alkyl chain contains 1 to 15 carbon atoms and the aryl group is as above described. Preferably, R is an alkyl or cycloalkyl group containing 4 to 12 carbon atoms or an alkaryl group in which the aromatic moiety is phenyl and the alkyl group is straight or branched chain alkyl or cycloalkyl containing up to 6 carbon atoms.

Examples of hydroperoxides for such decomposition are t-butyl and isobutyl hydroperoxide, isoamyl hydroperoxide, t-amylhydroperoxide, cyclohexyl hydroperoxide, alpha- and beta-ethylbenzene hydroperoxide, cumyl hydroperoxide, phenethyl hydroperoxide and cyclohexylphenyl hydroperoxide; phenethyl hydroperoxide and cumyl hydroperoxide are converted to phenethyl alcohol and cumyl alcohol respectively. Preferred are the alkyl hydroperoxides such as t-butyl hydroperoxide, isoamyl hydroperoxide, and the like, and the cycloalkyl hydroperoxides such as cyclohexanol, methylcyclohexanol hydroperoxide, and the like.

### SOLVENTS AND DECOMPOSITION CONDITIONS

The decomposition of hydroperoxides using the catalysts of the invention is typically carried out in a solution of the hydroperoxide in the solvent or solvent mixture. The solution preferably contains from about 5 to about 50wt% of hydroperoxide. Suitable solvents or co-solvents include benzene, chlorobenzene, o-dichlorobenzene, acetonitrile, benzonitrile, alcohols, ketones and the like. A useful solvent is the alcohol formed by decomposition of the hydroperoxide, for example TBA formed by decomposition of TBHP. Any suitable temperature and pressure may be used. Preferably the temperature is in the range from about 25 to about 130°C., and the total pressure from 0 to 500 psig; preferably, the partial pressure of the hydroperoxide in the solution is not more than about 50 psig. The time of reaction may be relatively short, in view of the rapid reaction rate with the catalysts employed according to the invention, and will typically be in the range from about 0.1 to about 5 hours.

The phthalocyanines according to the invention are useful as catalysts for the decomposition of hydroperoxides and for the partial oxidation of hydrocarbons, such as the oxidation of methane to methanol, the oxidation of isobutane to t-butanol, and the like.

### EXAMPLES

### Example 1

### Synthesis of Fe[(t-butyl)₄Pc]Cl

To a stirring solution of 6.5g of butylphthalic anhydride in 25ml of 1,2,4-trichlorobenzene is added 7.0g of urea, 0.2g of ammonium molybdate and finally 2.55g of anhydrous FeCl₃. The stirred mixture is heated to 190°C. and held there for 4hr after which time the solution is cooled and filtered. To the filtrate is added 25ml of petroleum ether (60-90°). After overnight this material is filtered to give dark blue microcrystalline material. The yield is 35% based on the starting t-butylphthalic anhydride, of Fe[(t-butyl)₄Pc)Cl. UV/vis(CHCl₃) 608,644 sh. 676 mm.

### Example 2

### Synthesis of Fe[(t-butyl)₄Pc]N₃

1.0g of the Fe[t-butyl)₄Pc]Cl is stirred in 20ml of acetone with 1.0g of NaN₃ for 12hr. The material is filtered and the filtrate evaporated to dryness. The solids are redissolved in chloroform and washed with H₂O then dried over sodium sulfate and evaporated to dryness. The yield is quantitative of Fe[(t-butyl)₄Pc]N₃ with a ν N-N in the IR spectrum of 2050 cm⁻¹.

### Example 3

The product of Example 4 was used as a catalyst for the decomposition of t-butylhydroperoxide. The catalyst was slurried in 3.9ml of acetone. The slurry was added to a solution of 13.8 grams of TBHP in 18.1 grams of TBA stirred at 80°C. The amount of catalyst was such as to give a solution containing 57ppm of catalyst. The solution was stirred at 78°C. for 144 hours. Oxygen evolution was followed manometrically and the liquid product profile monitored by standardized glpc. 1345 cc's of oxygen were evolved in the first hour of the reaction. All oxygen evolution had ceased after 0.4 hour, at which time there was 95% conversion of TBHP.

### Example 4

### Synthesis of Fe(FPc)-O-Fe(FPc)

A quantity of 300mg of iron perfluorophthalocyanine, Fe(FPc), is stirred in 100ml of tetrahydrofuran for 12 days in the presence of air. After this time the mixture is filtered through a medium coarse glass fritted funnel and the filtrate evaporated to dryness then heated in vacuo overnight at 110°C. The UV/vis spectrum shows a λₘₐₓ at 628nm. The mass spectrum shows a signal at M=1728, corresponding to this µ-oxo dimer.

### Example 5

The product of Example 1 was used as a catalyst for the decomposition of t-butylhydroperoxide. The catalyst was added to a solution of 13.8 grams of TBHP in 18.1 grams of TBA stirred at 80°C in a boat. The amount of catalyst was such as to give a solution containing 19 ppm of catalyst. Oxygen evolution was followed manometrically and the liquid product profile monitored by standardized glpc. 1160 cc's of oxygen were evolved in the first hour of the reaction. All oxygen evolution had ceased after 1 hour. The initial rate constant for the reaction was 0.17 for a run in which the TBHP and TBA had not been dried prior to use as catalyst, and 0.33 for a run in which the TBHP and TBA had been dried prior to use as catalyst.

## Claims

1. Compositions of matter having the formula: 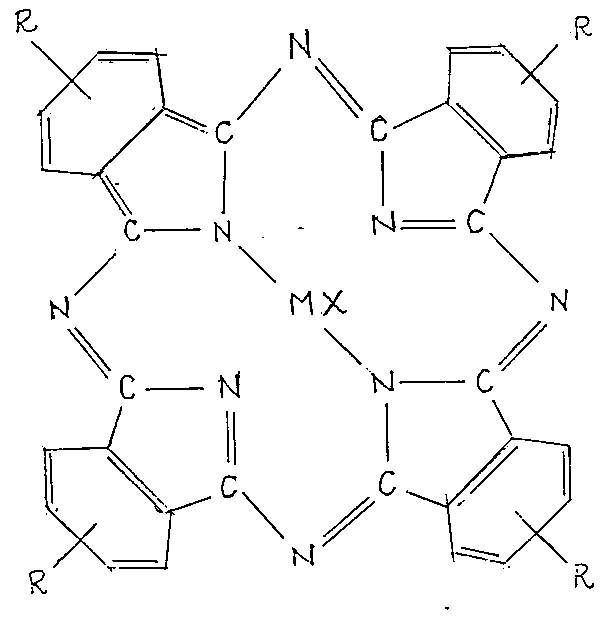 where (1) each R comprises, independently, one to four hydrocarbyl, halocarbyl or halohydrocarbyl groups, or hydrogen, at least one of said R's not being hydrogen, M comprises iron, cobalt, manganese, copper, ruthenium, or chromium, and X comprises halogen, hydroxyl or azide, or (2) said composition comprises a µ-oxo dimer of metallophthalocyanines having said formula.

2. Compositions according to claim 1 wherein R comprises hydrocarbyl.

3. Compositions according to claim 1 wherein R comprises t-butyl.

4. Composition according to claim 3 wherein said composition comprises Fe[(t-butyl)₄Pc]N₃, where Pc comprises phthalocyanine.

5. Compositions of matter comprising µ-oxo dimers comprising the formula: 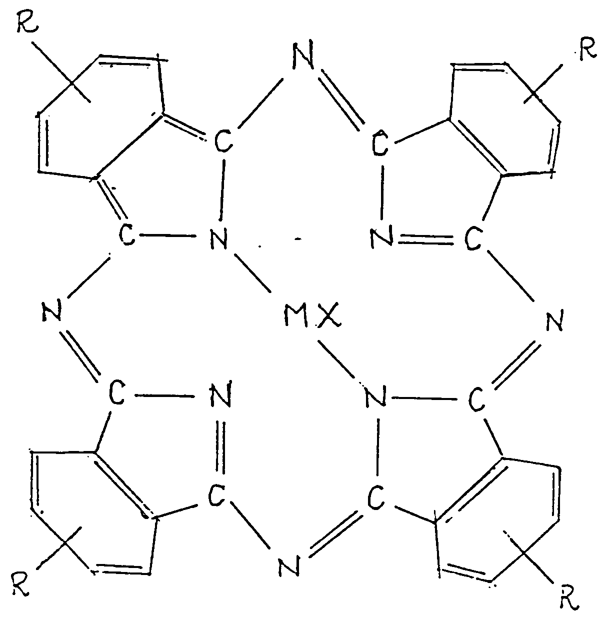 where each R comprises, independently, one to four halogen atoms, or hydrogen, at least one of said R's not being hydrogen, M comprises iron, cobalt, manganese, copper, ruthenium or chromium, and X comprises a µ-oxo linkage to a second metallophthalocyanine having said formula.

6. Compound according to claim 5, having the formula:
(XPc)M-O-M(XPc) where XPc represents perhalophthalocyanine.

7. Composition according to claim 5 comprising the compound, bis[perfluorophthalocyanatoiron(III)], Fe(FPc)-O-Fe(FPc), where FPc comprises perfluorophthalocyanine.

8. Use of a compound according to any of claims 1 to 7 as a catalyst for the decomposition of hydroperoxides and/or for the partial oxidation of hydrocarbons.
